# EUROPÄISCHE PATENTSCHRIFT

(11) **EP 0 514 415 B1**
(45) Veröffentlichungstag und Bekanntmachung des Hinweises auf die Patenterteilung: **06.05.1998**
(21) Anmeldenummer: 91903275.5
(22) Anmeldetag: 31.01.1991
(51) Int. Cl.: C09K 3/30, A61K 9/72

(54) **NEUE TREIBGASMISCHUNGEN UND IHRE VERWENDUNG IN ARZNEIMITTELZUBEREITUNGEN**
NOVEL VEHICLE GAS MIXTURES AND THEIR USE IN MEDICAL PREPARATIONS
NOUVEAUX MELANGES DE GAZ PROPULSEURS ET LEUR UTILISATION DANS DES PREPARATIONS MEDICAMENTEUSES

(30) Priorität: 03.02.1990 DE 4003272
(43) Veröffentlichungstag der Anmeldung: 25.11.1992
(73) Patentinhaber: BOEHRINGER INGELHEIM KG, 55216 Ingelheim (DE); BOEHRINGER INGELHEIM INTERNATIONAL GmbH, 55218 Ingelheim am Rhein (DE)
(72) Erfinder: WEIL, Hans-Hermann, D-6551 Gau-Bickelheim (DE); DAAB, Ottfried, D-6507 Ingelheim am Rhein (DE)
(86) Internationale Anmeldenummer: EP9100177
(87) Internationale Veröffentlichungsnummer: WO9111495

(56) Entgegenhaltungen:
- EP-A- 0 247 608
- EP-A- 0 384 371
- WO-A-86/04233
- WO-A-87/07502

## Beschreibung

Die Erfindung betrifft neue Treibgasmischungen, in denen als typischer Bestandteil 1,1,1,2,3,3,3-Heptafluorpropan (TG 227) und daneben 1,1,1,2-Tetrafluorethan, Pentafluorethan, TG 11, TG 12 oder TG 114 enthalten sind, die Verwendung dieser Treibgasmischungen in Arzneimittelzubereitungen, die zur Erzeugung von Aerosolen geeignet sind, sowie diese Arzneimittelzubereitungen selbst.

Aerosole von pulverförmigen (mikronisierten) Arzneistoffen werden vielfach in der Therapie, z.B. in der Therapie von obstruktiven Atemwegserkrankungen, eingesetzt. Soweit solche Aerosole nicht durch Zerstäuben des Arzneipulvers oder durch Versprühen von Lösungen erzeugt werden, benutzt man Suspensionen der Arzneistoffe in verflüssigten Treibgasen. Als solche dienen hauptsächlich Mischungen aus TG 11 (Trichlorfluormethan), TG 12 (Dithlordifluormethan) und TG 114 (1,2-Dichlor-1,1,2,2-tetrafluorethan), ggf. unter Zusatz von niederen Alkanen, etwa Butan, Pentan oder auch von DME (Dimethylether). Mischungen solcher Art sind beispielsweise aus der deutschen Patentschrift 1178975 bekannt.
Wegen ihres negativen Einflusses auf die Erdatmosphäre (Zerstörung der Ozonschicht, Treibhauseffekt) ist der Einsatz der Chlorfluorkohlenwasserstoffe zu einem Problem geworden, so daß nach anderen Treibgasen bzw. Treibgasmischungen gesucht wird, von denen die genannten negativen Wirkungen nicht oder wenigstens in geringerem Maß ausgehen.
Die Suche stößt jedoch auf erhebliche Schwierigkeiten, weil Treibgase, die therapeutisch eingesetzt werden sollen, zahlreiche Kriterien zu erfüllen haben, die nicht leicht in Einklang miteinander zu bringen sind, etwa hinsichtlich Toxizität, Stabilität, Dampfdruck, Dichte, Löseverhalten.
Wie nun gefunden wurde, sind aus zwei oder mehr Komponenten bestehende Treibgasmischungen, die mindestens TG 227 sowie eine oder mehrere Verbindungen aus der Gruppe TG 11, TG 12, TG 114, 1,1,1,2 Tetraflüorethan (TG 134a) und Pentafluorethan (TG 125), für den Einsatz in therapeutisch anwendbaren Zubereitungen besonders geeignet.

Zur Optimierung der Eigenschaften des Treibgasgemischs können Zusätze der bisher hauptsächlich verwendeten Treibgase TG 11, TG 12 und TG 114 nützlich sein, weil sie eine relativ hohe Dichte aufweisen. Arzneimittelzubereitungen, die auf der Basis der neuen Treibgasmischungen hergestellt werden, enthalten neben dem Wirkstoff (z.B. in suspendierter Form) im allgemeinen einen für diese Zwecke gebräuchlichen oberflächenaktiven Stoff, etwa einen Ester eines Polyalkohols, etwa einen Sorbitanester mit höheren gesättigten oder ungesättigten Fettsäuren, z.B. Sorbitantrioleat, oder ein Polyethoxysorbitanester einer höheren, vorzugsweise ungesättigten Fettsäure oder ein Phospholipid, etwa ein Lecithin. Der Hilfsstoff kann in der Mischung gelöst oder ungelöst vorliegen.

Um das Sedimentieren suspendierter Arzneistoffteilchen hintanzuhalten, ist es günstig, solche Mischungen der verflüssigten Treibgase zu verwenden, deren Dichte sich nicht erheblich von der Dichte des suspendierten Stoffes unterscheidet. Jedoch sind auch Mischungen mit größeren Dichteunterschieden zwischen Arzneistoff und verflüssigtem Treibgasgemisch brauchbar. Es hat sich nämlich gezeigt, daß entmischte Suspensionen durch Schütteln leicht wieder im hier vorgeschlagenen Suspensionsmedium gleichmäßig verteilt werden können.

Die Mengenverhältnisse der einzelnen Mischungsbestandteile des Treibgases können in weiten Grenzen variert werden. Der Anteil (jeweils in Gewichtsprozent) beträgt für TG 227 30 bis 95 %, für TG 125 20 bis 75 %, für TG 134a 20 bis 75 %, für TG 152a 25 bis 80 %. Die Mischung kann darüber hinaus 0 bis 50 % Propan und/oder Butan und/oder Pentan und/oder DME und 0 bis 25 % TG 11, TG 12 und/oder TG 114 enthalten. Innerhalb der genannten Grenzen werden die Bestandteile so gewählt, daß sich insgesamt 100 % ergeben.

Der Anteil an suspendiertem Arzneistoff an der fertigen Zubereitung beträgt zwischen 0,001 und 5 %, vorzugsweise 0,005 bis 3 %, insbesondere 0,01 bis 2 %. Die oberflächenaktiven Stoffe werden in Mengen von 0,01 bis 10 %, vorzugsweise 0,05 bis 5 %, insbesondere 0,1 bis 3 % zugegeben (hier wie bei den Arzneistoffen sind Gewichtsprozent der fertigen Zubereitung angegeben). Als Arzneistoffe in den neuen Zubereitungen können alle Substanzen dienen, die für die inhalative, ggf. auch für die intranasale Anwendung geeignet sind. Es handelt sich demnach insbesondere um Betamimetika, Anticholinergika, Steroide, Antiallergika,

PAF-Antagonisten sowie Kombinationen aus solchen Wirkstoffen.

Im einzelnen seien als Beispiele genannt:

Als Betamimetika:
Bambuterol
Bitolterol
Carbuterol
Clenbuterol
Fenoterol
Hexoprenalin
Ibuterol
Pirbuterol
Procaterol
Reproterol
Salbutamol
Salmeterol
Sulfonterol
Terbutalin
Tulobuterol

1-(2-Fluor-4-hydroxyphenyl)-2-[4-(1-benzimidazolyl)-2-methyl-2-butylamino]ethanol
erythro-5'-Hydroxy-8'-(1-hydroxy-2-isopropylaminobutyl)-2H-1,4-benzoxazin-3-(4H)-on
1-(4-Amino-3-chlor-5-trifluormethylphenyl)-2-tert.-butylamino)ethanol
1-(4-Ethoxycarbonylamino-3-cyan-5-fluorphenyl)-2-(tert.-butylamino)ethanol.

Als Anticholinergika:
Ipratropiumbromid
Oxitropiumbromid
Trospiumchlorid
Benzilsäure-N-β-fluorethylnortropinestermethobromid

Als Steroide:
Budesonid
Beclometason (bzw. das 17, 21-Dipropionat)
Dexamethason-21-isonicotinat
Flunisolid

Als Antiallergika:
Dinatriumcromoglicat
Nedocromil

Als PAF-Antagonisten:
WEB 2086
WEB 2170
WEB 2347

Die Wirkstoffe können auch kombiniert werden, z.B. Betamimetika plus Anticholinergika oder Betamimetica plus Antiallergika.

Beispiele für erfindungsgemäße Zubereitungen (Angabe in Gewichtsprozent):

| | | |
|---|---|---|
| 1.) | 0,3 % | Fenoterol |
| | 0,1 % | Sojalecithin |
| | 10,0 % | Pentan |
| | 70,0 % | TG 227 |
| | 19,6 % | TG 134a |

| | | |
|---|---|---|
| 2.) | 0,1 % | Ipratropiumbromid |
| | 0,1 % | Sojalecithin |
| | 25,0 % | Pentan |
| | 10,1 % | TG 227 |
| | 64,7 % | TG 134a |

| | | |
|---|---|---|
| 3.) | 0,3 % | Fenoterol |
| | 0,1 % | Sojalecithin |
| | 30,0 % | TG 11 |
| | 49,6 % | TG 134a |
| | 20,0 % | TG 227 |

| | | |
|---|---|---|
| 4.) | 0,3 % | Salbutamol |
| | 0,2 % | Span 85 |
| | 20,0 % | Pentan |
| | 30,0 % | TG 227 |
| | 49,5 % | TG 134a |

| | | |
|---|---|---|
| 5.) | 0,15 % | Fenoterol |
| | 0,06 % | Ipratropium- bromid |
| | 0,10 % | Sojalecithin |
| | 40,00 % | TG 11 |
| | 39,69 % | TG 134a |
| | 20,00 % | TG 227 |

| | | |
|---|---|---|
| 6.) | 0,1 % | Ipratropium-bromid |
| | 0,1% | Sojalecithin |
| | 20,3 % | TG 125 |
| | 25,5 % | % TG 152a |
| | 54,0 % | TG 227 |

## Patentansprüche

1. Treibgasmischungen aus zwei oder mehr Komponenten für inhalative pharmazeutische Zubereitungen, dadurch gekennzeichnet, daß sie 1,1,1,2,3,3,3-Heptafluorpropan und daneben eine oder mehrere Verbindungen aus der Gruppe 1,1,1,2-Tetrafluorethan, Pentafluorethan, TG 11, TG 12 oder TG 114 enthalten, wobei der Gehalt an 1,1,1,2,3,3,3-Heptafluorpropan zwischen 30 und 95 Gewichtsprozent liegt.

2. Treibgasmischungen nach Anspruch 1, dadurch gekennzeichnet, daß sie zusätzlich mindestens einen oberflächenaktiven Stoff enthalten.

3. Treibgasmischungen nach Anspruch 2, dadurch gekennzeichnet, daß der oberflächenaktive Stoff ein Phospholipid, ein Sorbitanester mit einer höheren gesättigten oder ungesättigten Fettsäure oder ein Polyethoxysorbitanester einer höheren, vorzugsweise ungesättigten Fettsäure ist.

4. Treibgasmischungen nach Anspruch 2, dadurch gekennzeichnet, daß der oberflächenaktive Stoff ein Lecithin, ein Polyoxyethylensorbitanoleat oder Sorbitantrioleat ist.

5. Inhalative Arzneimittelzubereitungen zur Erzeugung von Pulveraerosolen auf der Basis von Treibgasmischungen nach Anspruch 1, 2, 3 oder 4, dadurch gekennzeichnet, daß sie als Wirkstoff ein Betamimetikum, ein Anticholinergikum, ein Steroid, ein Antiallergikum oder einen PAF-Antagonisten oder eine Kombination solcher Verbindungen enthalten.

6. Arzneimittelzubereitungen nach Anspruch 5, dadurch gekennzeichnet, daß
als Betamimetikum
Bambuterol
Bitolterol
Carbuterol
Clenbuterol
Fenoterol
Hexoprenalin
Ibuterol
Pirbuterol
Procaterol
Reproterol
Salbutamol
Salmeterol
Sulfonterol
Terbutalin
Tulobuterol
1-(2-Fluor-4-hydroxyphenyl)-2-[4-(1-benzimidazolyl)-2-methyl-2-butylamino]ethanol
erythro-5'-Hydroxy-8'-(1 -hydroxy-2-isopropylamino-butyl)-2H-1,4-benzoxazin-3-(4H)-on
1-(4-Amino-3-chlor-5-trifluormethylphenyl-butyl-amino)ethanol
1 -(4-Ethoxycarbonylamino-3-cyan-5-fluorphenyl)-2-(tert.-butylamino)ethanol
als Anticholinergika:
Ipratropiumbromid
Oxitropiumbromid
Trospiumchlorid
Benzilsäure-N-β-fluorethylnortropinestermethobromid
als Steroide:
Budesonid
Beclometason (bzw. das 17, 21-Dipropionat)
Dexamethason-21-isonicotinat
Flunisolid
als Antiallergikum:
Dinatriumcromoglicat
Nedocromil
als PAF-Antagonisten:
4-(2-Chlorphenyl)-9-methyl-2-[3-(4-morpholinyl)-3-propanon-1-yl]-6H-thieno[3,2-f][1,2,4]triazolo[4,3-a][1,4]diazepin
6-(2-Chlorphenyl)-8,9-dihydro-1-methyl-8-[4-morpholinyl)carbonyl]-4H-7H-cyclopenta[4,5]thieno[3,2-f][1,2,4]triazolo[4,3-a][1,4]diazepin
6-(2-Chlorphenyl)-8,9-dihydro-1-methyl-8-(N,N-dipropyl-aminocarbonyl)-4H-7H-cyclopenta[4,5]thieno[3,2-f][1,2,4]triazolo[4,3-a][1,4]diazepin
verwendet wird.

7. Arzneimittelzubereitungen nach Anspruch 5, dadurch gekennzeichnet, daß die Kombination der Wirkstoffe eines der in Anspruch 6 genannten Betamimetika und eines der in Anspruch 6 genannten Anticholinergika umfaßt.

8. Arzneimittelzubereitungen nach Anspruch 5, dadurch gekennzeichnet, daß die Kombination der Wirkstoffe eines der in Anspruch 6 genannten Betamimetika und Dinatriumcromoglic umfaßt.

9. Arzneimittelzubereitungen nach Anspruch 5, dadurch gekennzeichnet, daß die Kombination der Wirkstoffe eines der in Anspruch 6 genannten Betamimetika und einen der in Anspruch 6 genannten PAF-Antagonisten enthält.

10. Arzneimittelzubereitungen nach Anspruch 5, dadurch gekennzeichnet, daß die Kombination der Wirkstoffe Dinatriumcromoglicat und einen der in Anspruch 6 genannten PAF-Antagonisten umfaßt.

## Claims

1. Propellant gas mixtures made from two or more components for inhaled pharmaceutical preparations, characterised in that they contain 1,1,1,2,3,3,3-heptafluoropropane and, in addition, one or more compounds from the group 1,1,1,2-tetrafluoroethane, pentafluoroethane, TG 11, TG 12 or TG 114, where the quantity of 1,1,1,2,3,3,3-heptafluoropropane is between 30 and 95% by weight.

2. Propellant gas mixtures according to claim 1, characterised in that they additionally contain at least one surface-active substance.

3. Propellant gas mixtures according to claim 2, characterised in that the surface-active substance is a phospholipid, a sorbitan ester with a higher saturated or unsaturated fatty acid or a polyethoxysorbitan ester of a higher, preferably unsaturated, fatty acid.

4. Propellant gas mixture according to claim 2, characterised in that the surface-active substance is a lecithin, a polyoxyethylene sorbitan oleate or a sorbitan trioleate.

5. Inhaled pharmaceutical preparations for the production of powder aerosols based on propellant gas mixtures according to claims 1, 2, 3 or 4, characterised in that they contain a betamimetic, an anticholinergic, a steroid, an antiallergic or a PAF-antagonist as their active ingredient, or that they contain a combination of such compounds.

6. Pharmaceutical preparations according to claim 5, characterised in that the betamimetic used is:
Bambuterol
Bitolterol
Carbuterol
Clenbuterol
Fenoterol
Hexoprenaline
Ibuterol
Pirbuterol
Procaterol
Reproterol
Salbutamol
Salmeterol
Sulphonterol
Terbutaline
Tulobuterol
1-(2-fluoro-4-hydroxyphenyl)-2-[4-(1-benzimidazolyl)-2-methyl-2-butylamino]ethanol
Erythro-5'-Hydroxy-8'-(1-hydroxy-2-isopropylaminobutyl)-2H-1,4-benzoxazine-3-(4H)-one
1-(4-amino-3-chloro-5-trifluoromethylphenyl)-2-tert.-butyl-amino)ethanol
1-(4-ethoxycarbonylamino-3-cyano-5-fluorophenyl)-2-(tert.-butylamino)ethanol
the anticholinergic used is:
Ipratropium bromide
Oxitropium bromide
Trospium chloride
Benzilic acid N-β-fluorethylnortopine ester
methobromide
the steroid used is:
Budesonide
Beclomethasone (or the 17,21-dipropionate)
Dexamethazone-21-isonicotinate
Flunisolide
the antiallergic used is:
Disodium cromoglycate
Nedocromil
the PAF-antagonist used is:
4-(2-chlorophenyl)-9-methyl-2-[3-(4-morpholinyl)-3-propanone-1-yl]-6H-thieno[3,2-f] [1,2,4]triazolo [4,3-a] [1,4]diazepine
6-(2-chlorophenyl)-8,9-dihydro-1-methyl-8[4-(morpholinyl)carbonyl]-4H-7H-cyclopenta[4,5]thieno[3,2-f] [1,2,4]triazolo[4,3-a][1,4]diazepine
6-(2-chlorophenyl)-8,9-dihydro-1-methyl-8-(N,N-dipropyl-aminocarbonyl)-4H-7H-cyclopenta[4,5]thieno[3,2-f] [1,2,4]triazolo[4,3-1][1,4]diazepine

7. Pharmaceutical preparations according to claim 5, characterised in that the combination of active ingredients comprises one of the betamimetics specified in claim 6 and one of the anticholinergics specified in claim 6.

8. Pharmaceutical preparations according to claim 5, characterised in that the combination of active ingredients comprises one of the betamimetics specified in claim 6 and disodium cromoglycate.

9. Pharmaceutical preparations according to claim 5, characterised in that the combination of active ingredients comprises one of the betamimetics specified in claim 6 and one of the PAF-antagonists specified in claim 6.

10. Pharmaceutical preparations according to claim 5, characterised in that the combination of active ingredients contains disodium cromoglycate and one of the PAF-antagonists specified in claim 6.

## Revendications

1. Mélanges de gaz propulseurs constitués par deux ou plusieurs composants pour préparations pharmaceutiques pour inhalation, caractérisés en ce qu'ils contiennent du 1,1,1,2,3,3,3-heptafluoropropane et en outre un ou plusieurs composés du groupe du 1,1,1,2-tétrafluoroéthane, du pentafluoroéthane, de TG 11, de TG 12 ou de TG 114, la teneur en 1,1,1,2,3,3,3-heptafluoropropane étant située entre 30 et 95 % en masse.

2. Mélanges de gaz propulseurs selon la revendication 1 caractérisés en ce qu'ils contiennent en outre au moins une substance tensioactive.

3. Mélanges de gaz propulseurs selon la revendication 2 caractérisés en ce que la substance tensioactive est un phospholipide, un ester de sorbitane et d'un acide gras supérieur saturé ou insaturé ou un ester de polyéthoxysorbitane et d'un acide gras supérieur, de préférence insaturé.

4. Mélanges de gaz propulseurs selon la revendication 2 caractérisés en ce que la substance tensioactive est une lécithine, un oléate de polyoxyéthylénesorbitane ou le trioléate de sorbitane.

5. Préparations médicamenteuses pour inhalation pour la production d'aérosols de poudres à base de mélanges de gaz propulseurs selon la revendication 1, 2, 3 ou 4 caractérisées en ce qu'elles contiennent comme principe actif un bêta-mimétique, un anticholinergique, un stéroïde, un antiallergique ou un antagoniste du PAF, ou une combinaison de tels composés.

6. Préparations médicamenteuses selon la revendication 5 caractérisées en ce que l'on utilise comme bêta-mimétique :
le bambutérol
le bitoltérol
le carbutérol
le clenbutérol
le fénotérol
l'hexoprénaline
l'ibutérol
le pirbutérol
le procatérol
le reprotérol
le salbutamol
le salmétérol
le sulfontérol
la terbutaline
le tulobutérol
le 1-(2-fluoro-4-hydroxyphényl)-2-[4-(1-benzimidazolyl)-2-méthyl-2-butylamino]éthanol
l'érythro-5'-hydroxy-8'-(1-hydroxy-2-isopropylaminobutyl)-2H-1,4-benzoxazin-3-(4H)-one
le 1-(4-amine-3-chloro-5-trifluorométhylphényl)-2-tert.butylamino)éthanol
le 1-(4-éthoxycarbonylamino-3-cyano-5-fluorophinyl)-2-(tert.butylamino)éthanol
comme anticholinergiques:
le bromure d'ipratropium
le bromure d'oxitropium
le chlorure de trospium
le méthobromure d'ester d'acide benzilique et de N-β-fluoroéthylnortropine
comme stéroïdes:
le budésonide
la béclométasone (ou le 17,21-dipropionate)
le 21-isonicotinate de dexaméthasone le flunisolide
comme antiallergique:
le cromoglycate disodique
le nédocromil
comme antagonistes du PAF:
la 4-(2-chlorophenyl)-9-méthyl-2-[3-(4-morpholinyl)-3-propanon-l-yl]-6H-thiéno[3,2-f][1,2,4]triazolo[4,3-a]-[1,4]diazépine
la 6-(2-chlorophényl)-8,9-dihydro-1-méthyl-8-[(4-morpholinyl)carbonyl]-4H-7H-cyclopenta[4,5]thiéno[3,2-f]-[1,2,4]triazolo[4,3-a][1,4]diazipine
la 6-(2-chlorophényl)-8,9-dihydro-1-méthyl-8-(N,N-dipropyl-aminocarbonyl)-4H-7H-cyclopenta[4,5]thiéno-[3,2-f][1,2,4]triazolo[4,3-a][1,4]diazépine.

7. Préparations médicamenteuses selon la revendication 5 caractérisées en ce que la combinaison des principes actifs comprend l'un des bêta-mimétiques cités dans la revendication 6 et l'un des anticholinergiques cités dans la revendication 6.

8. Préparations médicamenteuses selon la revendication 5 caractérisées en ce que la combinaison des principes actifs comprend l'un des bêta-mimétiques cités dans la revendication 6 et du cromoglycate disodique.

9. Préparations médicamenteuses selon la revendication 5 caractérisées en ce que la combinaison des principes actifs contient l'un des bêta-mimétiques cités dans la revendication 6 et l'un des antagonistes du PAF cités dans la revendication 6.

10. Préparations médicamenteuses selon la revendication 5 caractérisées en ce que la combinaison des principes" actifs comprend du cromoglycate disodique et l'un des antagonistes du PAF cités dans la revendication 6.
